(19) Europäisches Patentamt
European Patent Office
Office européen des brevets

(11) EP 3 889 144 A1

(12) EUROPEAN PATENT APPLICATION

(43) Date of publication:
**06.10.2021 Bulletin 2021/40**

(51) Int Cl.:
***C07D 309/10** (2006.01)*

(21) Application number: **21169098.7**

(22) Date of filing: **09.10.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.10.2016 EP 16193735**

(62) Document number(s) of the earlier application(s) in accordance with Art. 76 EPC:
**17777935.2 / 3 526 229**

(71) Applicant: **Boehringer Ingelheim International GmbH**
**55216 Ingelheim am Rhein (DE)**

(72) Inventors:
- **WIRTH, Thomas**
  **55216 Ingelheim am Rhein (DE)**
- **BERG, Alexander**
  **55216 Ingelheim am Rhein (DE)**
- **MEYNHARDT, Bernd**
  **55216 Ingelheim am Rhein (DE)**
- **WEBER, Dirk**
  **55216 Ingelheim am Rhein (DE)**

(74) Representative: **Simon, Elke Anna Maria et al**
**Boehringer Ingelheim International GmbH**
**Corporate Patents**
**Binger Strasse 173**
**55216 Ingelheim am Rhein (DE)**

Remarks:
This application was filed on 19-04-2021 as a divisional application to the application mentioned under INID code 62.

(54) **PROCESS FOR PREPARING GLUCOPYRANOSYL-SUBSTITUTED BENZYL-BENZENE DERIVATIVES**

(57) The present invention relates to processes for preparing glucopyranosyl-substituted benzylbenzene derivatives of general formula III,

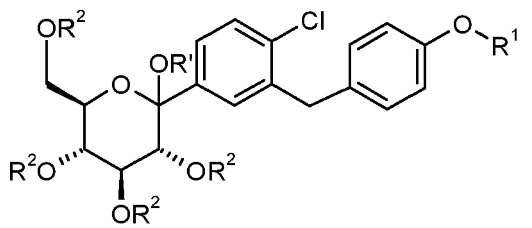

III

wherein $R^1$, $R^2$ and R' are defined according to claim 1;
and the use of such processes in the synthesis of SGLT2 inhibitors.

EP 3 889 144 A1

## Description

Field of the Invention

The present invention relates to processes for preparing glucopyranosyl-substituted benzyl-benzene derivatives of the formula III,

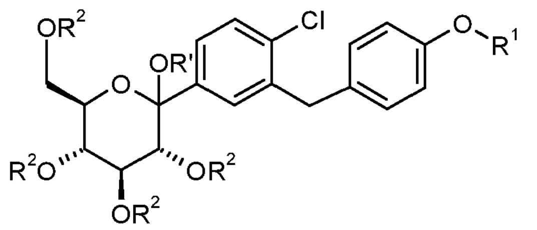

III

wherein the substituents $R^1$, $R^2$ and R' are defined as hereinafter.

In addition, the present invention relates to the use of the processes according to the invention, e.g. for the synthesis of inhibitors of the sodium-dependent glucose cotransporter SGLT2.

Background of the Invention

In WO 2005/092877, glucopyranosyl-substituted benzene derivatives of the general formula

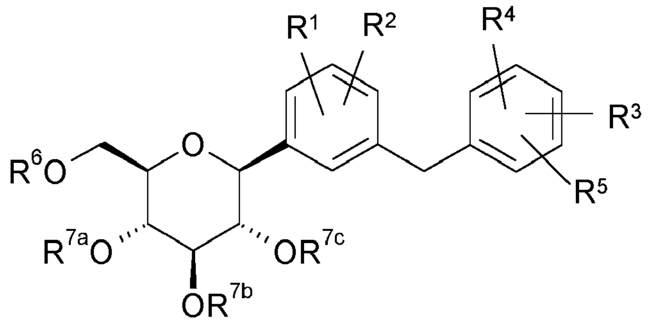

are described wherein the groups $R^1$ to $R^6$ and $R^{7a}$, $R^{7b}$, $R^{7c}$ are as defined therein.

In WO 2006/117359, a crystalline form of 1-chloro-4-(β-*D*-glucopyranos-1-yl)-2-[4-((*S*)-tetrahydrofuran-3-yloxy)-benzyl]-benzene and its synthesis are described.

In WO 2006/120208, several methods of synthesis of compounds of the general formula

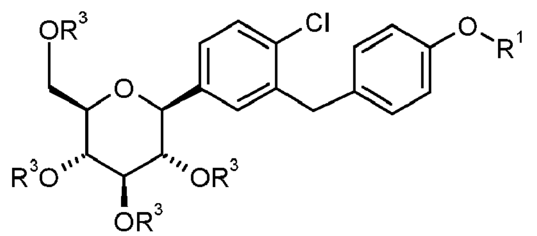

are described wherein $R^1$ denotes, among others, R-tetrahydrofuran-3-yl and S-tetrahydrofuran-3-yl and $R^3$ is as defined therein. The example XVIII therein relates to the synthesis of 1-chloro-4-(β-*D*-glucopyranos-1-yl)-2-(4-(*S*)-tetrahydrofuran-3-yloxy-benzyl)-benzene.

In WO 2011/039108, modified processes are described for preparing glucopyranosyl-substituted benzyl-benzene derivatives of the general formula

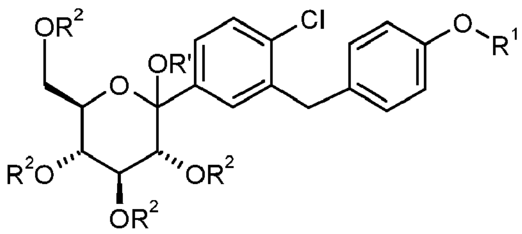

wherein $R^1$ denotes, among others, (*R*)-tetrahydrofuran-3-yl and (*S*)-tetrahydrofuran-3-yl and R' and $R^2$ are as defined therein. In these processes, the C-C bond between the glycoside and the aglycone is formed in step (S2) by reaction of a gluconolactone with an organometallic species, for instance an aryl Grignard compound.

It is known, however, that aryl Grignard reagents are prone to homo-coupling reactions, in particular in the presence of transition metal salts. This can be exploited preparatively (Kharasch et al., J. Am. Chem. Soc. 1941, 63, 2316.), but may also be observed as an unwanted side reaction in cross-couplings (Fürstner et al., J. Am. Chem. Soc. 2002, 124, 13856.).

Object of the Invention

The object of the present invention is to provide advantageous processes for preparing a glucopyranosyl-substituted benzyl-benzene derivative of formula III,

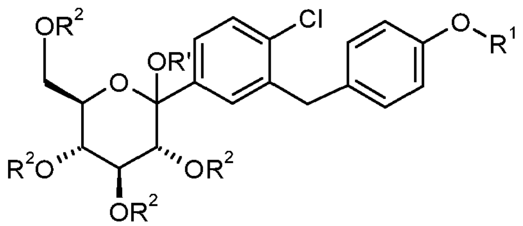

III

wherein $R^1$, $R^2$ and R' are defined as hereinafter;
in particular processes conducted under conditions to reduce side reactions that may impact the yield and the impurity profile of the substance obtained by the process.

In particular, an object of the present invention is to provide a process in which unwanted side reactions are reduced by carrying out the process up to and including the C-C bond forming step at sufficiently low concentrations of iron ions, in particular by choosing appropriate qualities of the equipment and purities of the reagents employed.

A further object of the present invention is to provide the use of the above-mentioned processes for the synthesis of a compound of formula IV

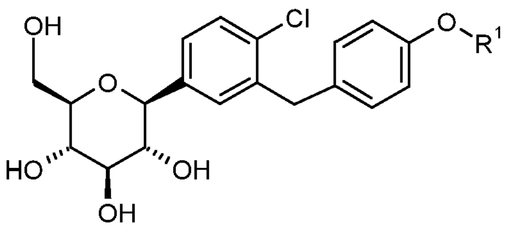

IV

wherein $R^1$ is defined as hereinafter.

Other objects of the present invention will become apparent to the person skilled in the art directly from the foregoing and following description.

In a first aspect, the present invention relates to a process for preparing a glucopyranosyl-substituted benzyl-benzene derivative of general formula III,

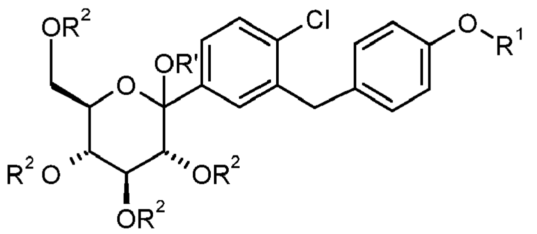

III

wherein

$R^1$ denotes (R)-tetrahydrofuran-3-yl or (S)-tetrahydrofuran-3-yl; and

$R^2$ independently of one another denote hydrogen, ($C_{1-8}$-alkyl)carbonyl-, ($C_{1-8}$-alkyl)oxycarbonyl-, phenylcarbonyl-, phenyl-($C_{1-3}$-alkyl)-carbonyl-, phenyl-$C_{1-3}$-alkyl-, allyl-, $R^aR^bR^cSi$, $CR^aR^bOR^c$, wherein two adjacent groups $R^2$ may be linked with each other to form a bridging group $SiR^aR^b$, $CR^aR^b$ or $CR^aOR^b$-$CR^aOR^b$; and wherein $R^a$, $R^b$, $R^c$ independently of one another denote $C_{1-4}$-alkyl, phenyl or phenyl-$C_{1-3}$-alkyl-, while the alkyl groups may be mono- or polysubstituted by halogen; while the phenyl groups mentioned in the definition of the above groups may be mono- or polysubstituted with L1, wherein L1 independently of one another are selected from among fluorine, chlorine, bromine, $C_{1-3}$-alkyl, $C_{1-4}$-alkoxy and nitro; and

R' denotes hydrogen, methyl or ethyl;

comprising the steps (S1), (S2) and (S3):

(S1): reacting a compound of general formula I

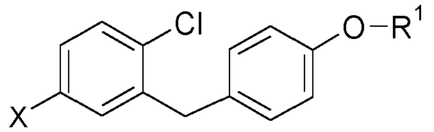

I

wherein $R^1$ is defined as hereinbefore and X denotes Br, I or triflate;
with a $C_{1-4}$-alkyl-magnesium chloride or bromide,
wherein lithium bromide and/or lithium chloride is optionally used, and

(S2): reacting the organometallic compound obtained in step (S1) with a compound of general formula II

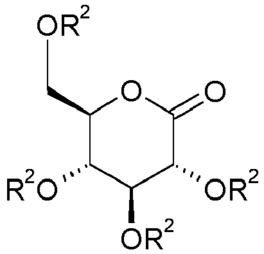

II

wherein $R^2$ is defined as hereinbefore; and
wherein lithium bromide and/or lithium chloride is optionally used, and
wherein $R^2$ not being hydrogen are optionally cleaved during or at the end of (S2), and

(S3): reacting the adduct obtained in step (S2) with a compound R'-OH or a mixture of compounds R'-OH, wherein R' is defined as hereinbefore, in the presence of one or more acids,

characterized in that,
the mole ratio of iron ions in the reaction mixtures of step (S1) and/or step (S2) to compound I employed in step (S1) does not exceed 40 ppm.

In a second aspect, the present invention relates to the use of the above-mentioned process for preparing a compound of general formula III in the synthesis of a compound of general formula IV

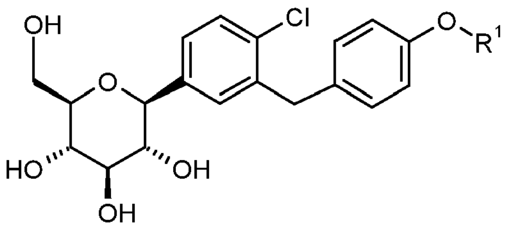

IV

wherein $R^1$ is defined as hereinbefore;
comprising step (S4) and optionally comprising step (S5):

(S4): reacting the compound of general formula III with a reducing agent; and optionally

(S5): cleavage of the protective groups $R^2$ not being hydrogen in the compound formed in step (S4).

Detailed Description of the Invention

In the following, the process steps relevant to this invention are described; they are disclosed in detail in WO 2006/120208 and WO 2011/039108.

Unless otherwise stated, the groups, residues and substituents, particularly $R^1$, $R^2$, $R^a$, $R^b$, $R^c$, R', L1, X, are defined as hereinbefore and hereinafter.

If residues, substituents or groups occur several times in a compound, they may have the same or different meanings.

In the processes according to this invention, the following meanings of groups and substituents are preferred:

$R^1$ preferably denotes (S)-tetrahydrofuran-3-yl.
$R^2$ preferably denotes hydrogen, methylcarbonyl, ethylcarbonyl or trimethylsilyl. Most preferably, $R^2$ denotes trimethylsilyl.
$R^a$, $R^b$, $R^c$ independently of one another preferably denote methyl, ethyl, n-propyl, iso-propyl, tert-butyl or phenyl; most preferably methyl.
R' preferably denotes methyl.
X preferably denotes I.

Any and each of the above definitions of the substituents may be combined with one another.

An overview of the reaction steps according to the present invention that lead to the formation of a compound of general formula III is given in Scheme 1: The glucopyranosyl-substituted benzyl-benzene derivative of formula III may be synthesized by the reaction of D-gluconolactone or a derivative thereof (II) with the desired benzyl-benzene compound in the form of an organometallic compound Ib.

Scheme 1: Overview of the reaction steps for the synthesis of compound III

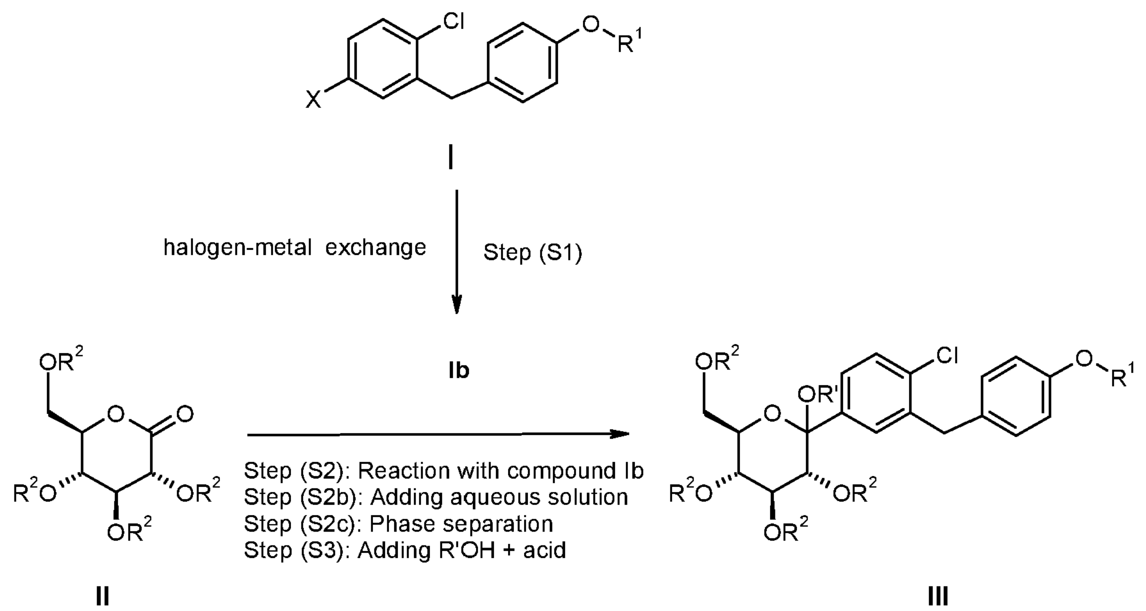


The starting materials for the processes according to the invention, i.e. the compound of formula I and the gluconolactone of formula II, may be synthesized according to the procedures disclosed in WO 2011/039108 (see compounds of formula V and IV, respectively, therein).

The process according to the invention comprises step (S1), a halogen-metal exchange reaction, in which the organometallic compound (Ib) is prepared by reacting the compound of formula I

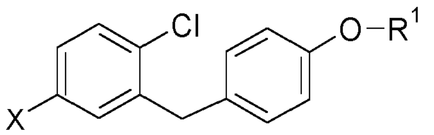

I

with a magnesium Grignard reagent in an organic medium.

The Grignard reagent is preferably a $C_{1-4}$-alkyl-magnesium chloride or bromide, more preferably a $C_{3-4}$-alkyl-magnesium chloride or bromide, most preferably isopropyl magnesium chloride. Optionally, lithium chloride and/or lithium bromide, preferably lithium chloride, may be used, e.g. as promoters, at the beginning of, during or at the end of step (S1). Most preferably, a mixture of isopropyl magnesium chloride and lithium chloride is employed.

In the following, the term "Grignard reagent" shall be used for $C_{1-4}$-alkyl-magnesium chloride and/or bromide, optionally in admixture with lithium chloride and/or bromide. Solutions comprising the Grignard reagent, preferably with tetrahydrofuran (THF), 2-methyltetrahydrofuran or a mixture thereof as the solvent, shall be meant by the term "Grignard solution" (GriS).

Suitable conditions and means (e.g. mole ratios, solvents, further additives, temperatures, reaction times, atmospheric conditions) for carrying out and monitoring the reaction are detailed in WO 2011/039108 or are known to the one skilled in the art.

In particular, the reaction is preferably conducted under the following conditions: The most preferred Grignard reagent is a mixture of isopropyl magnesium chloride and lithium chloride. Most preferably, the Grignard reagent is employed in the form of a solution in tetrahydrofuran. The mole ratio of isopropyl magnesium chloride and lithium chloride is preferably in the range from 1 : 10 to 10 : 1, most preferably about 1 : 1. The most preferred amount of the Grignard reagent relative to the compound of formula I is in range from about 0.5 : 1 to 2 : 1 most preferably about equimolar. Most preferably, the reaction is carried out in THF or 2-methyl-THF or a mixture thereof. The most preferred temperature range is from -40°C to -10°C and the preferred reaction time between 10 min and 600 min. Preferably, the reaction is performed under argon and/or nitrogen inert gas atmosphere. The reaction product of step (S1), the organometallic compound Ib may be isolated, although such an isolation is not necessary.

In step (S2), the gluconolactone of formula II is added to the organometallic compound Ib in an organic medium, preferably to the reaction mixture obtained in step (S1).

Optionally, lithium chloride and/or lithium bromide, preferably lithium chloride, may be used, e.g. as promoters, at the beginning of, during or at the end of step (S2).
Suitable conditions and means (e.g. mole ratios, solvents, temperatures, reaction times, atmospheric conditions) for carrying out and monitoring the reaction and workup procedures are detailed in WO 2011/039108 or are known to the one skilled in the art.
In particular, the reaction is preferably conducted under the following conditions: Preferably, the reaction is carried out in tetrahydrofuran or 2-methyltetrahydrofurane or a mixture thereof. The preferred amount of the gluconolactone II relative to the organometallic compound Ib is about 1 : 1 to 2 : 1, most preferably about 1.06 : 1. The most preferred temperature range is from -20°C to -5°C and the preferred reaction time between 15 min and 600 min. Preferably, the reaction is performed under argon and/or nitrogen inert gas atmosphere.
The reaction product may be isolated.

In step (S2b), an acidic aqueous solution is added to the reaction mixture obtained in step (S2) such that the reaction mixture forms an aqueous phase and an organic phase whereby the organic phase has a pH in the range from about 0 to 7.
Suitable conditions and means (e.g. acids, acid concentrations, volume ratios, temperatures, addition times, additional salts, additional organic solvents, distillation) for achieving phase separation and measuring the pH value are detailed in WO 2011/039108 or are known to the one skilled in the art.
In particular, the following conditions are preferred: The pH range in the organic phase is preferably from about 1 to 4, most preferably from about 2 to 3. The pH value is measured preferably at a temperature between about 10°C and 30°C. Preferred acids for the aqueous solution are citric acid, acetic acid and tartaric acid, most preferred is citric acid. The acid concentration ranges preferably from 5 to 20 weight-%, most preferably it is about 10 weight-%. The volume of the aqueous solution relative to the volume of the reaction mixture obtained in the step (S2) is most preferably in the range from about 0.3 to 0.6, for example about 0.4. The aqueous solution is added to the reaction mixture most preferably at a temperature from about 10°C to 25°C, most preferably within at least 60 min. Advantageously and most preferably, the volume of the organic phase is reduced by distillation under reduced pressure at a temperature below or equal to about 35°C and further amounts of 2-methyl¬tetrahydrofurane are added, most preferably about 15 to 35 weight-% relative to the total organic phase of the reaction mixture.
Additionally, depending on the nature of $R^2$, cleavage of $R^2$ not being hydrogen may be optionally effected by the reaction conditions applied during step (S2b).

In step (S2c), the organic phase comprising most of the adduct obtained in step (S2) and/or (S2b) is separated from the aqueous phase. The aqueous phase may be washed with an organic medium and the organic phases may be combined. Preferably, the volume of the organic phase is reduced by distillation prior to the next reaction step.
Suitable conditions and means (e.g. solvents, temperature, pressure) for separation of the liquid phases and distillation are detailed in WO 2011/039108 or are known to the one skilled in the art.
In particular, the phase separation is performed most preferably at temperatures from about 0°C to 30°C and the organic solvents are distilled off, preferably under reduced pressure and at temperatures below or equal to 35°C.

In step (S3), the adduct obtained in the preceding steps is reacted with a compound R'-OH or a mixture of compounds R'-OH, wherein R' denotes hydrogen, methyl or ethyl, preferably methyl, in the presence of one or more acids, preferably in the presence of hydrochloric acid. A full conversion to the product of formula III is advantageously achieved by a subsequent distillation. After the completion of the reaction, the remaining acid in the reaction mixture is preferably neutralized by the addition of one or more bases, most preferably triethylamine. A partial or the total amount of the solvent is preferably distilled off.
Suitable conditions and means (e.g. amounts, pH values, temperatures, times, distillation parameters, reduction of water content) for carrying out the reaction and characterizing the product are detailed in WO 2011/039108 or are known to the one skilled in the art.
In particular, the reaction is preferably conducted under the following conditions: The alcohol is preferably employed in an amount exceeding an equimolar amount. By the addition of the one or more acids, a preferred pH value between about 0 and 4, most preferably between 1 and 2 is obtained. The preferred reaction temperature is between 15°C and 25°C and the preferred reaction time is up to 120 min. The distillation is preferably carried out at reduced pressure and at a temperature below or equal about 35°C. The preferred pH range after neutralization is in the range from 5 to 6. Most preferably, the solvent is distilled off at reduced pressure, acetonitrile is added and distilled off again and dichloromethane is added as a solvent.
Additionally, depending on the nature of $R^2$, cleavage of $R^2$ not being hydrogen may optionally be effected by the reaction conditions applied during step (S3).

A compound of formula IV may be synthesized via step (S4), a reduction of the anomeric carbon-oxygen bond of compound III, and via optional step (S5), the cleavage of $R^2$ not being hydrogen (Scheme 2).

Scheme 2: Reduction and optional deprotection of compound III

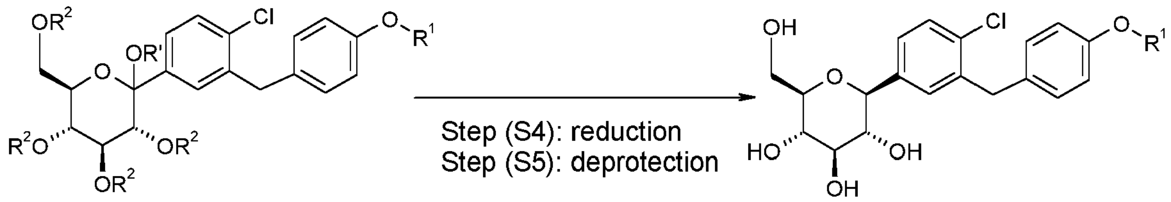


III

IV

$R^1$, $R^2$ and R' are defined as hereinbefore. A preferred meaning of $R^2$ is hydrogen or trimethylsilyl. R' preferably denotes hydrogen, methyl or ethyl, most preferably methyl. In step (S4), the reduction may be conducted in an organic medium with one or more reducing agents, preferably triethylsilane, in the presence of one or more Lewis acids, preferably aluminium chloride, or without a Lewis acid.

Alternatively, in step (S4), hydrogen may be used as reducing agent in the presence of a transition metal catalyst.

Suitable conditions and means (e.g. amounts, reducing reagents, Lewis acids, solvents, temperatures, times, atmospheric conditions) for carrying out the reaction and workup procedures are detailed in WO 2011/039108 or are known to the one skilled in the art.

In particular, the reaction is preferably conducted under the following conditions: Preferably the reaction mixture obtained in step (S4) is added to a mixture of one or more organic solvents, the one or more reducing agents and the one or more Lewis acids. The preferred molar amount of the reducing agent relative to compound III is about 2 : 1 to 4 : 1, most preferably about 2.7 : 1. The preferred molar amount of the Lewis acid agent relative to compound III is about 2 : 1 to 4 : 1, most preferably about 2.1 : 1. Most preferred solvents for the reaction are acetonitrile, dichloromethane or mixtures thereof. The preferred reaction temperature is between about 0°C and 30°C, most preferably between 10°C and 20°C. The reaction components are added preferably within 45 min to 120 min and the mixture is preferably stirred for about 30 min to 120 min at about 0°C to 35°C, most preferably at about 15°C to 25°C. Preferably, the reaction is performed under argon and/or nitrogen inert gas atmosphere.

Additionally, depending on the nature of $R^2$, cleavage of $R^2$ not being hydrogen may optionally be effected by the reaction conditions applied during step (S4).

In an optional step (S5), the protective groups $R^2$ not being hydrogen are cleaved from the compound obtained in step (S4), resulting in the compound of formula IV.

Suitable conditions for achieving this depend on the nature of $R^2$, but are detailed in WO 2011/039108 or are known to the one skilled in the art.

The product may be obtained by crystallisation, for example as described in WO 2006/117359 or WO 2011/039108.

It was found that the performance of this process is particularly sensitive to the presence of iron ions, in particular in steps (S1) and (S2): With increasing iron ion concentrations, the formation of oligomers of I and the like was observed so that the yield and the impurity profile of the obtained product are impaired.

This effect was demonstrated experimentally by adding different levels of iron ions to Grignard solutions (isopropyl magnesium chloride and lithium chloride in tetrahydrofuran) to be used in the process according to the invention. This was performed either via direct spiking of iron salts (in order to simulate iron ion impurities present in the reaction mixtures) or by adding pre-treated metal test pieces (in order to simulate the release of iron ions from reactor materials into the solution).

The amount of iron ions was investigated by means of ICP-MS. At the end of step (S1), the amount of oligomers formed was determined via HPLC-UV. At the end of step (S2), the amount of the actually desired hemiacetal product (compound of formula III wherein R' denotes H) was measured by HPLC-UV. The results of these investigations are summarized in the section "Description and Results of Experimental Procedures".

The spiking experiments revealed that even iron ion mass fractions (e.g. $Fe^{2+}$ and/or $Fe^{3+}$) in the low single-digit ppm range in the Grignard solution promote the formation of oligomers of I and the like to a substantial degree and largely suppress the formation of the desired hemiacetal intermediate.

Thus, according to one embodiment of the present invention, the mole ratio of iron ions in the reaction mixtures of step (S1) and/or (S2) to compound I employed in step (S1) does not exceed 40 ppm, preferably 30 ppm, most preferably 20 ppm.

According to another embodiment of the present invention, the mole ratio of iron ions in the reaction mixtures of steps (S1) and/or (S2) to alkyl-magnesium species employed in step (S1) does not exceed 40 ppm, preferably 30 ppm, most

preferably 20 ppm.
According to another embodiment of the present invention, the mole ratio of iron ions in the reaction mixture of step (S2) to compound II employed in step (S2) does not exceed 40 ppm, preferably 30 ppm, most preferably 20 ppm.
According to another embodiment of the present invention, the mass fraction of iron ions in the reaction mixtures of steps (S1) and/or (S2) does not exceed 1.5 ppm, preferably 1.1 ppm, most preferably 0.75 ppm.

As a consequence, reagents, in particular Grignard solutions, with very low iron ion concentrations are advantageously employed in the process of the invention.
Thus, according to one embodiment of the present invention, the mole ratio of iron ions in the Grignard solution to $C_{1-4}$-alkyl-magnesium species in the Grignard solution does not exceed 40 ppm, preferably 30 ppm, most preferably 20 ppm.
According to another embodiment of the present invention, the mass fraction of iron ions in the Grignard solution employed in step (S1) does not exceed 3 ppm, preferably 2.2 ppm, most preferably 1.5 ppm.

As a further potential source of iron ions, different reactor materials were tested for the process of the invention (see section "Description and Results of Experimental Procedures"); they were in fact found to be able to release iron ions to different extents when corrosion or oxidation processes were simulated by pre-treatment of the metal test pieces. Such corrosion or oxidation processes are common and well known events in dedicated or multi-purpose chemical manufacturing equipment (e.g. reactors, tubing, containers etc.) and may be induced or accelerated by corrosive agents (e.g. hydrochloric acid) and the presence of oxygen. Corrosive agents (e.g. hydrochloric acid) and oxygen are abundant in any dedicated or multi-purpose chemical manufacturing plant. Another factor influencing these corrosion processes is the type or quality of the construction materials used for the reactors, tubing and containers. The above described corrosion processes can lead to leaching of iron ions into the reaction mixtures of steps (S1) and/or (S2), as defined hereinbefore, resulting in iron ion mass fractions above 0.75 ppm and the formation of oligomers of I.

Therefore, according to another embodiment of the present invention, the process of the invention is carried out in equipment in which the materials of the surfaces that may come into contact with the Grignard solution and/or with the reaction mixtures of steps (S1) and/or (S2), in particular the materials of those surfaces that are in contact with the reaction mixtures during the performance of the reactions, are resistant against releasing or leaching of iron ions into the reaction mixtures under the reaction conditions of steps (S1) and/or (S2) described hereinbefore and hereinafter. The above-mentioned resistance to releasing or leaching of iron ions shall mean that the above-mentioned criteria for mass fractions and mole ratios of iron ions in the Grignard solution and in the reaction mixtures of steps (S1) and/or (S2) are met.
Thus, preferably, the materials of said surfaces are selected from the group consisting of metal alloys, in particular nickel alloys, with iron mass fractions of not more than 10%, preferably of not more than 6%, most preferably of not more than 1.5%. Non-limiting examples of such metal alloys are Alloy 22 (2.4602) with a typical Fe mass fraction of up to 6% and Alloy 59 (2.4605) with a typical Fe mass fraction of up to 1.5%.
According to another embodiment of the invention, the materials of said surfaces are selected from the group consisting of materials that are treated and/or coated to prevent releasing or leaching of iron ions. Non-limiting examples are glass-lined, metal-plated or polymer-coated surfaces, e.g. glass-lined steel.

**Description and Results of Experimental Procedures:**

**Experiment A**

Pre-Treatment of Grignard solution (GriS; *i*-PrMgCl / LiCl in THF):

In a glass flask, to a 1.3 mol/L solution of *i*-PrMgCl / LiCl in THF (100 mL) the respective iron salt ($FeI_2$ or $FeCl_3$) was spiked and the resulting mixture was stirred at room temperature for 7 days under argon atmosphere. Then, a sample was taken and analyzed for the iron ion content with analytical method A.

Description of the Experiment:

In a glass flask, a solution of compound I, wherein X denotes I and $R^1$ denotes (S)-tetrahydrofuran-3-yl, (0.072 mol) in THF (54 mL) was cooled to -15 °C to -40 °C under argon atmosphere. 55 mL of the pre-treated Grignard solution (1.0 eq) were added at -15 °C to -40 °C within 60-65 min. A sample was taken and analyzed for compound I and oligomers with analytical method B and C, respectively. To this solution, compound II, wherein $R^2$ denotes trimethylsilyl, (1.1 eq) was added at -5 °C to -25 °C. After completion of the addition, the resulting mixture was stirred at -5 °C to -15 °C for additional 60-120 min. A sample was taken and analyzed for the hemiacetal intermediate of formula III (R' = H) with analytical method D.

**Table 1: Results of Experiment A**

| Spiked iron salt | Mass fraction w(Fe) in GriS [ppm] (method A) | Mole ratio r (Fe/Mg) [ppm][2] | Amount of unreacted I [area%] (method B) | Amount of oligomers of I [area%] (method C) | Amount of hemiacetal III [area%] (method D) |
|---|---|---|---|---|---|
| no spiking[1] | *not applicable* | *not applicable* | 3.9 | 0.7 | 82.0 |
| $FeI_2$ | 4.0 | 54 | 10.2 | 60.8 | 1.8 |
| $FeCl_3$ | 10.0 | 135 | 46.1 | 70.3 | *not detected* |

[1] reference experiment
[2] calculated from mass fraction w(Fe) in Grignard solution (see analytical method A)

**Experiment B**

Pre-Treatment of the metal test piece:

The respective metal test piece was stored in a desiccator under an atmosphere of 5M aqueous hydrochloric acid for 4 weeks.

Pre-Treatment of Grignard solution (GriS; *i*-PrMgCl / LiCl in THF):

In a glass flask, to a 1.3 mol/L solution of *i*-PrMgCl / LiCl in THF (100 mL) the respective pre-treated metal test piece was added and the resulting mixture was stirred at room temperature for 7 days under argon atmosphere. Then, a sample was taken and analyzed for the iron ion content with analytical method A.

Description of the Experiment:

In a glass flask, a solution of compound I, wherein X denotes I and $R^1$ denotes (*S*)-tetrahydrofuran-3-yl, (0.072 mol) in THF (54 mL) was cooled to -15 °C to -40 °C under argon atmosphere. 55 mL of the pre-treated Grignard solution (1.0 eq) was added at -15 °C to -40 °C within 60-65 min. A sample was taken and analyzed for compound I and oligomers with analytical method B and C, respectively. To this solution, compound II, wherein $R^2$ denotes trimethylsilyl, (1.1 eq) was added at -5 °C to -25 °C. After completion of the addition, the resulting mixture was stirred at -5 °C to -15 °C for additional 60-120 min. A sample was taken and analyzed for the hemiacetal intermediate of formula III (R' = H) with analytical method D.

**Table 2: Results of Experiment B**

| Spiked metal test piece | Mass fraction w (Fe) in GriS [ppm] (method A) | Mole ratio r (Fe/Mg) [ppm][2] | Amount of unreacted I [area%] (method B) | Amount of oligomers of I [area%] (method C) | Amount of hemiacetal III [area%] (method D) |
|---|---|---|---|---|---|
| no spiking[1] | *not applicable* | *not applicable* | 3.9 | 0.7 | 82.0 |
| Alloy 59 (2.4605) | < 1.5 | < 20 | 7.2 | 23.5 | 67.8 |
| Stainless steel A4L (1.4404) | 19 | 256 | 71.3 | 50.4 | *not detected* |
| Stainless steel V2A (1.4301) | 15 | 202 | 68.1 | 59.4 | *not detected* |
| flat steel (P265GH) | 228 | 3078 | 81.6 | 28.3 | *not detected* |

[1] reference experiment
[2] calculated from mass fraction w(Fe) in Grignard solution (see analytical method A)

**Description of Analytical Methods:**

**Analytical Method A**

For the quantification of iron ion concentrations, a quantitative analytical method using ICP-MS (e.g. Perkin Elmer Nexion 300) was used. Samples were filtered using membrane filters (e.g. Pall Acrodisc Premium 25 mm Syringe Filter 0.45 μm GHP Membrane) and were, after addition of nitric acid and hydrochloric acid, digested using a microwave (e.g. Anton Paar Multiwave 3000). Iron ion amounts in solution are determined as mass fractions w(Fe), i.e. the mass of iron ions divided by the mass of the solution, and are given in this document as ppm, i.e. μg (Fe) / g (solution).

The mass fraction of iron ions in the Grignard solution (w(Fe)) may be converted into the mole ratio of iron ions to organomagnesium species (r(Fe/Mg), i.e. the molar amount of iron ions divided by the molar amount of organomagnesium species) with the help of the following formula:

$$r\left(\frac{Fe}{Mg}\right) = \frac{n(Fe)}{n(Mg)} = \frac{w(Fe)}{c(Mg)} * \frac{\rho(GriS)}{M(Fe)}$$

wherein p(GriS) means the density of the Grignard solution (980 g/L), c(Mg) the molar concentration of the Grignard solution (1.3 mol/L) and M(Fe) the molar mass of iron (55.845 g/mol). The mole ratios are given in ppm, i.e. μmol (Fe) / mol (Mg).

**Analytical Method B**

Reaction monitoring method: Gradient HPLC apparatus; eluent A: 1.0 mL trifluoroacetic acid dissolved in 1.0 L HPLC water; eluent B: 1.0 mL trifluoroacetic acid dissolved in 1.0 L gradient grade acetonitrile; HPLC column: Agilent, Zorbax Eclipse XDB-C8, 4.6*150 mm, particle size 5 μm; column temperature: 25 °C; flow: 2.0 mL/min; gradient profile: 0 min, 30% eluent A, 70% eluent B; 5 min, 20% eluent A, 80% eluent B; equilibration 5 min; sample preparation: direct quench of 0.1 mL reaction mixture with 10 mL methanol; injection volume: 1.0 μL; UV-detection: 230 nm; data evaluation: only peaks of compound I (X = I, $R^1$ = (S)-tetrahydrofuran-3-yl; retention time approx. 3.2 min) and quenched intermediate (compound I with X = H, $R^1$ = (S)-tetrahydrofuran-3-yl;, retention time approx. 2.2 min) are taken into account for area% calculation.

**Analytical Method C**

Oligomer monitoring method: Gradient HPLC apparatus; eluent A: 1.0 mL perchloric acid dissolved in 1.0 L HPLC water; eluent B: gradient grade acetonitrile; column: AMT Halo C8, 4.6*150 mm, particle size 2.7 μm; column temperature: 35 °C; flow: 1.5 mL/min; gradient profile: 0 min, 60% eluent A, 40% eluent B; 20 min, 10% eluent A, 90% eluent B; 25 min, 0% eluent A, 100% eluent B; 35 min, 0% eluent A, 100% eluent B; equilibration 5 min; sample preparation: direct quench of 0.1 mL reaction mixture with 10 mL methanol; dilute 500 μL of quenched solution with 500 μL THF; injection volume: 1.0 μL; UV-detection: 224 nm; data evaluation: all peaks in chromatogram are taken into account for area% calculation, peaks eluting later than compound I (X = I, $R^1$ = (S)-tetrahydrofuran-3-yl; retention time approx. 11.4 min) are summarized and reported as "oligomers of compound I".

**Analytical Method D**

Reaction monitoring method: Gradient HPLC apparatus; eluent A: 1.0 mL trifluoroacetic acid dissolved in 1.0 L HPLC water; eluent B: 1.0 mL trifluoroacetic acid dissolved in 1.0 L gradient grade acetonitrile; HPLC column: Agilent, Zorbax Eclipse XDB-C8, 4.6*150 mm, particle size 5 μm; column temperature: 25 °C; flow: 1.2 mL/min; gradient profile: 0 min, 70% eluent A, 30% eluent B; 7 min, 60% eluent A, 40% eluent B; 15 min, 5% eluent A, 95% eluent B; 30 min, 5% eluent A, 95% eluent B; equilibration 7 min; sample preparation: direct quench of 0.1 mL reaction mixture with 5 mL 1 N hydrochloric acid, dilute with 5 mL acetonitrile; injection volume: 1.0 μL; UV-detection: 230 nm; data evaluation: all peaks integrated for area% calculation; reported hemiacetal intermediate (compound of formula III wherein R' = H, $R^1$ = (S)-tetrahydrofuran-3-yl, $R^2$ = trimethylsilyl) at retention time approx. 3.9 min.

**Claims**

**1.** Process for preparing a compound of general formula III,

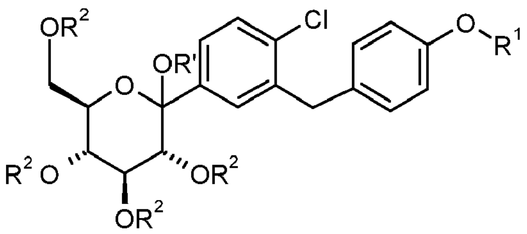

III

wherein $R^1$ denotes (*R*)-tetrahydrofuran-3-yl or (*S*)-tetrahydrofuran-3-yl; and wherein $R^2$ independently of one another denote hydrogen, ($C_{1-8}$-alkyl)carbonyl, ($C_{1-8}$-alkyl)oxycarbonyl, phenylcarbonyl, phenyl-($C_{1-3}$-alkyl)-carbonyl, phenyl-$C_{1-3}$-alkyl, allyl, $R^aR^bR^cSi$, $CR^aR^bOR^c$, wherein two adjacent groups $R^2$ may be linked with each other to form a bridging group $SiR^aR^b$, $CR^aR^b$ or $CR^aOR^b$-$CR^aOR^b$;

wherein $R^a$, $R^b$, $R^c$ independently of one another denote $C_{1-4}$-alkyl, phenyl or phenyl-$C_{1-3}$-alkyl, while the alkyl groups may be mono- or polysubstituted by halogen; while the phenyl groups mentioned in the definition of the above groups may be mono- or polysubstituted with L1, wherein L1 independently of one another are selected from among fluorine, chlorine, bromine, $C_{1-3}$-alkyl, $C_{1-4}$-alkoxy and nitro; and

wherein R' denotes hydrogen, methyl or ethyl; comprising the steps (S1), (S2) and (S3):

(S1): reacting a compound of general formula I

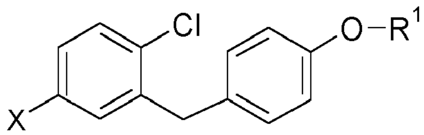

I

wherein $R^1$ is defined as hereinbefore and X denotes Br, I or triflate; with a $C_{1-4}$-alkyl-magnesium chloride or bromide, wherein lithium bromide and/or lithium chloride is optionally used, and

(S2): reacting the organometallic compound obtained in step (S1) with a compound of general formula II

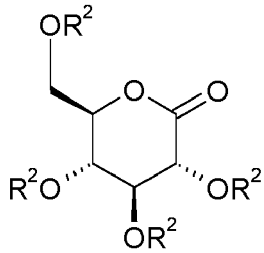

II

wherein $R^2$ is defined as hereinbefore; and wherein lithium bromide and/or lithium chloride is optionally used, and wherein $R^2$ not being hydrogen are optionally cleaved during or at the end of (S2), and

(S3): reacting the adduct obtained in step (S2) with a compound R'-OH or a mixture of compounds R'-OH, wherein R' is defined as hereinbefore, in the presence of one or more acids,

**characterized in that**, the mole ratio of iron ions in the reaction mixtures of step (S1) and/or (S2) to compound I employed in step (S1) does not exceed 40 ppm.

**2.** The process according to claim 1 wherein X in step (S1) denotes I.

**3.** The process according to any of claims 1 and 2 wherein $C_{3-4}$-alkyl-magnesium chloride or bromide, preferably isopropyl magnesium chloride is employed in step (S1) and additionally lithium chloride is used in step (S1).

**4.** The process according to any of claims 1 to 3 wherein $R^2$ denotes trimethylsilyl in the compound of general formula II used in step (S2).

**5.** The process according to any of claims 1 to 4 wherein R' in step (S3) denotes methyl.

**6.** Use of the process for preparing a compound of general formula III according to any of claims 1 to 5 in the synthesis of a compound of general formula IV

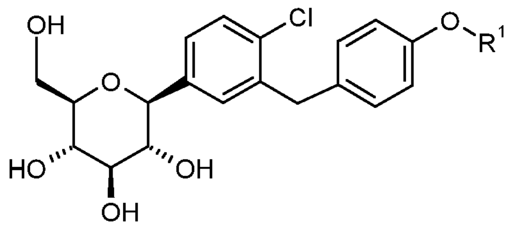

**IV**

wherein $R^1$ is defined as hereinbefore;
comprising step (S4) and optionally comprising step (S5):

(S4): reacting the compound of general formula III with a reducing agent; and optionally
(S5): cleavage of the protective groups $R^2$ not being hydrogen in the compound formed from the compound of general formula III in step (S4).

**7.** The process according to any of claims 1 to 6 wherein, in the reagent comprising the alkyl-magnesium species used in step (S1) and/or in a solution comprising such reagent, the mole ratio of iron ions to $C_{1-4}$-alkyl-magnesium species does not exceed 40 ppm.

**8.** The process according to any of claims 1 to 7 wherein the materials of the equipment surfaces that may come into contact with a solution comprising the alkyl-magnesium species used in step (S1) and/or with the reaction mixtures of steps (S1) and/or (S2) are resistant against releasing or leaching of iron ions such that the mole ratio of iron ions in the reaction mixtures of step (S1) and/or (S2) to compound I employed in step (S1) does not exceed 40 ppm.

**9.** The process according to any of claims 1 to 8 wherein the materials of the equipment surfaces that may come into contact with a solution comprising the alkyl-magnesium species used in step (S1) are resistant against releasing or leaching of iron ions such that, in said solution, the mole ratio of iron ions to $C_{1-4}$-alkyl-magnesium species does not exceed 40 ppm.

**10.** The process according to any of claims 1 to 9 wherein the materials of the equipment surfaces that may come into contact with a solution comprising the alkyl-magnesium species used in step (S1) and/or with the reaction mixtures of steps (S1) and/or (S2) are selected from the group consisting of metal alloys with iron mass fractions of not more than 10%.

**11.** The process according to any of claims 1 to 10 wherein the materials of the equipment surfaces that may come into contact with a solution comprising the alkyl-magnesium species used in step (S1) and/or the reaction mixtures of steps (S1) and/or (S2) are selected from the group consisting of materials that are treated and/or coated to prevent releasing or leaching of iron ions.

Europäisches Patentamt
European Patent Office
Office européen des brevets

# EUROPEAN SEARCH REPORT

Application Number
EP 21 16 9098

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2011/039108 A2 (BOEHRINGER INGELHEIM INT [DE]; WEBER DIRK [DE]; FIEDLER TOBIAS [DE]; F) 7 April 2011 (2011-04-07)<br>----- | 1-11 | INV.<br>C07D309/10 |
| A,D | WO 2006/120208 A1 (BOEHRINGER INGELHEIM INT [DE]; BOEHRINGER INGELHEIM PHARMA [DE]; ECKHA) 16 November 2006 (2006-11-16)<br>----- | 1-11 | |
| A,D | FUERSTNER A ET AL: "Iron-Catalyzed Cross-Coupling Reactions",<br>JOURNAL OF THE AMERICAN CHEMICAL SOCIETY, AMERICAN CHEMICAL SOCIETY, US,<br>vol. 124, 1 January 2002 (2002-01-01), pages 13856-13863, XP002250741,<br>ISSN: 0002-7863, DOI: 10.1021/JA027190T<br>----- | 1-11 | |

TECHNICAL FIELDS SEARCHED (IPC)

C07D

The present search report has been drawn up for all claims

2

| Place of search | Date of completion of the search | Examiner |
|---|---|---|
| Munich | 8 June 2021 | Klein, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 16 9098

This annex lists the patent family members relating to the patent documents cited in the above-mentioned European search report. The members are as contained in the European Patent Office EDP file on
The European Patent Office is in no way liable for these particulars which are merely given for the purpose of information.

08-06-2021

| Patent document cited in search report | Publication date | Patent family member(s) | Publication date |
|---|---|---|---|
| WO 2011039108 A2 | 07-04-2011 | AR 078193 A1 | 19-10-2011 |
| | | AU 2010303124 A1 | 15-03-2012 |
| | | BR 112012007085 A2 | 15-09-2015 |
| | | CA 2775962 A1 | 07-04-2011 |
| | | CL 2012000581 A1 | 03-08-2012 |
| | | CN 102574829 A | 11-07-2012 |
| | | DK 2486029 T3 | 24-08-2015 |
| | | EA 201200537 A1 | 30-11-2012 |
| | | EP 2486029 A2 | 15-08-2012 |
| | | ES 2546762 T3 | 28-09-2015 |
| | | HR P20150945 T1 | 09-10-2015 |
| | | HU E026133 T2 | 30-05-2016 |
| | | JP 5758900 B2 | 05-08-2015 |
| | | JP 2013505975 A | 21-02-2013 |
| | | KR 20120081592 A | 19-07-2012 |
| | | NZ 598366 A | 28-03-2014 |
| | | PL 2486029 T3 | 30-11-2015 |
| | | PT 2486029 E | 14-10-2015 |
| | | SI 2486029 T1 | 30-10-2015 |
| | | TW 201127848 A | 16-08-2011 |
| | | US 2011237789 A1 | 29-09-2011 |
| | | US 2015218200 A1 | 06-08-2015 |
| | | WO 2011039108 A2 | 07-04-2011 |
| WO 2006120208 A1 | 16-11-2006 | AR 057010 A1 | 07-11-2007 |
| | | AU 2006245712 A1 | 16-11-2006 |
| | | BR PI0608603 A2 | 19-01-2010 |
| | | CA 2606577 A1 | 16-11-2006 |
| | | CA 2835779 A1 | 16-11-2006 |
| | | CN 101193903 A | 04-06-2008 |
| | | CN 103524468 A | 22-01-2014 |
| | | EA 200702347 A1 | 28-04-2008 |
| | | EP 1881990 A1 | 30-01-2008 |
| | | HK 1116802 A1 | 02-01-2009 |
| | | IL 187229 A | 29-02-2012 |
| | | IL 215274 A | 31-07-2013 |
| | | JP 4834080 B2 | 07-12-2011 |
| | | JP 2008540489 A | 20-11-2008 |
| | | KR 20080017343 A | 26-02-2008 |
| | | NZ 564028 A | 29-07-2011 |
| | | SG 10201503667Y A | 29-06-2015 |
| | | TW I384981 B | 11-02-2013 |
| | | UA 99896 C2 | 25-10-2012 |
| | | US 2006258749 A1 | 16-11-2006 |
| | | US 2010240879 A1 | 23-09-2010 |
| | | US 2015322053 A1 | 12-11-2015 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 21 16 9098

This annex lists the patent family members relating to the patent documents cited in the above-mentioned European search report.
The members are as contained in the European Patent Office EDP file on
The European Patent Office is in no way liable for these particulars which are merely given for the purpose of information.

08-06-2021

| Patent document cited in search report | Publication date | Patent family member(s) | Publication date |
|---|---|---|---|
| | | WO 2006120208 A1 | 16-11-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005092877 A
- WO 2006117359 A
- WO 2006120208 A
- WO 2011039108 A


### Non-patent literature cited in the description


- **KHARASCH et al.** *J. Am. Chem. Soc.,* 1941, vol. 63, 2316
- **FÜRSTNER et al.** *J. Am. Chem. Soc.,* 2002, vol. 124, 13856